# EUROPÄISCHE PATENTANMELDUNG

(11) **EP 3 229 024 A1**
(43) Veröffentlichungstag der Anmeldung: **11.10.2017**
(21) Anmeldenummer: 16164573.4
(22) Anmeldetag: 08.04.2016
(51) Int. Cl.: G01N 33/569, G01N 33/543

(54) **ZIKA VIRUS DIAGNOSTIK**

(71) Anmelder: Viramed Biotech AG, 82152 Planegg (DE)
(72) Erfinder: Kronsteiner, Lilly, 82152 Planegg/Steinkirchen (DE); Kintrup, Martin, 82131 Gauting (DE); Scheide, Dierk, 82100 Germering (DE)
(74) Vertreter: Grünecker Patent- und Rechtsanwälte PartG mbB

(57) **Zusammenfassung**

Die vorliegende Erfindung betrifft Vorrichtungen und Methoden zur Durchführung von Experimenten zum Nachweis von spezifischen Wechselwirkungen zwischen Sonden- und Zielmolekülen, die auf eine Zika Virus Infektion rückschliessen lassen.

## Beschreibung

Die vorliegende Erfindung betrifft Vorrichtungen und Methoden zur Durchführung von Experimenten zum Nachweis von spezifischen Wechselwirkungen zwischen Sonden- und Zielmolekülen, die auf eine Zika Virus Infektion rückschliessen lassen.

Das Zika Virus (ZIKV) gehört zur Familie der Flaviviridae, zu der auch andere Pathogene wie Dengue Virus (DENV), West Nil Virus (WNV), Gelbfieber Virus (YFV), Frühsommermeningoenzephalitis Virus/ tick-borne encephalitic virus (TBEV), St. Louis Enzephalitis Virus (SLV) und Japanisches Enzephalitis Virus (JEV) gehören.

Das ZIKV wurde zuerst 1947 aus einem Rhesus Affen im Zika-Forest in Uganda isoliert (Dick et al., 1952). Ab 2007 wurde über etwas über 100 Infektionen im westlichen Pazifik in Mikronesien berichtet. 2013 wurde etwa ein Zehntel der Bevölkerung von Französisch-Polynesien mit dem Zika Virus infiziert. Seit 2015 treten millionenfach Zika Virus Infektionen in Süd- und Mittelamerika auf, insbesondere in Brasilien und Kolumbien. In geringem, jedoch zunehmendem Maße treten auch in den USA und in Europa Fälle von ZIKV Infektion auf, vor allem bei Reiserückkehrern.

Flaviviren sind umhüllte Viren, die ein RNA-Genom von etwa 11.000 bp in einem Kapsid enthalten. Das Kapsid besteht aus identischen Kapsid-(C-)Proteinen und ist umhüllt von 180 Kopien zweier Hüllproteine, nämlich des Envelope (E) Glykoproteins ( etwa 500 Aminosäuren) bzw. des Membran (M) Proteins (etwa 75 Aminosäuren) oder dessen Vorläufer Precursor Membran (prM) Proteins (Sirohi et al., Science 2016). Hierbei macht das E Protein den Großteil der Gesamtproteinmenge aus.

Neben diesen Strukturproteinen kodiert das Genom sieben weitere, sog. Nichtstruktur-Proteine (NS1, NS2A, NS2B, NS3, NS4A, NS4B, NS5), die an der Replikation und Assemblierung des Virus beteiligt sind und eine Rolle bei der Wechselwirkung mit dem Immunsystem des Wirts spielen.

Die Proteine werden in Form eines Polyproteins gebildet, dass anschließend proteolytisch gespalten wird (Li et al., 2008).

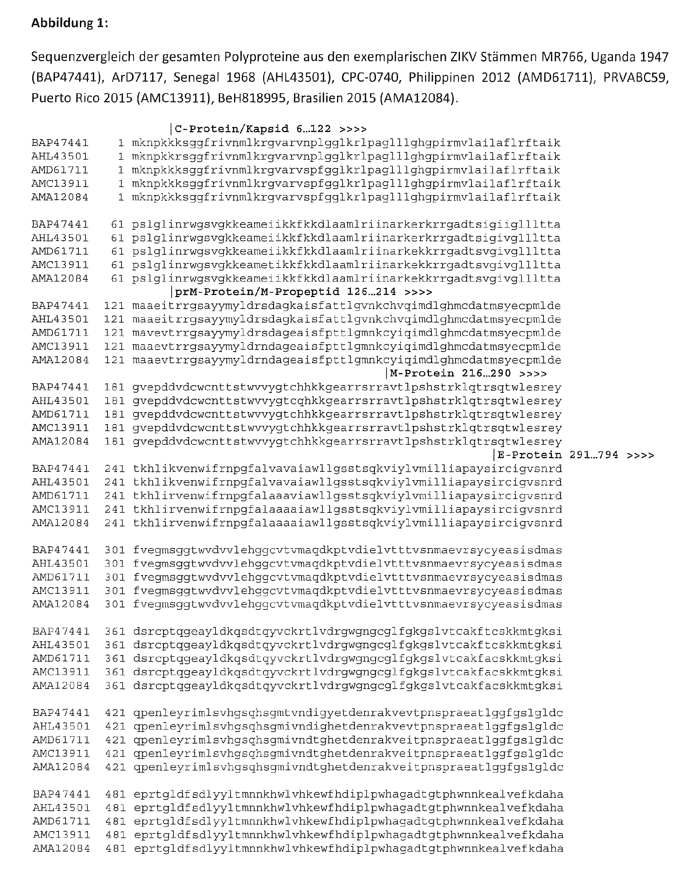

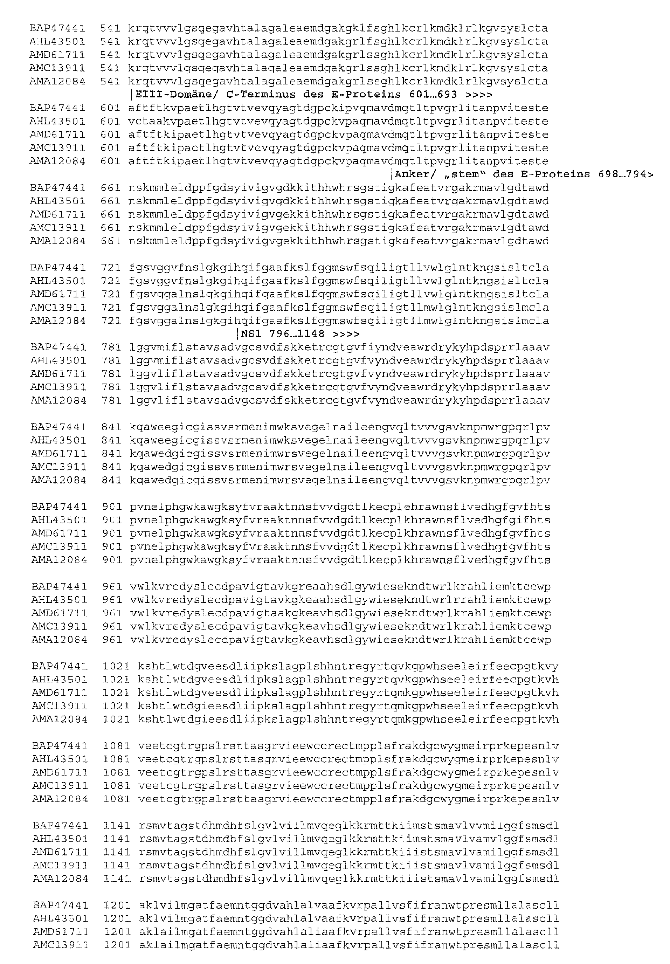

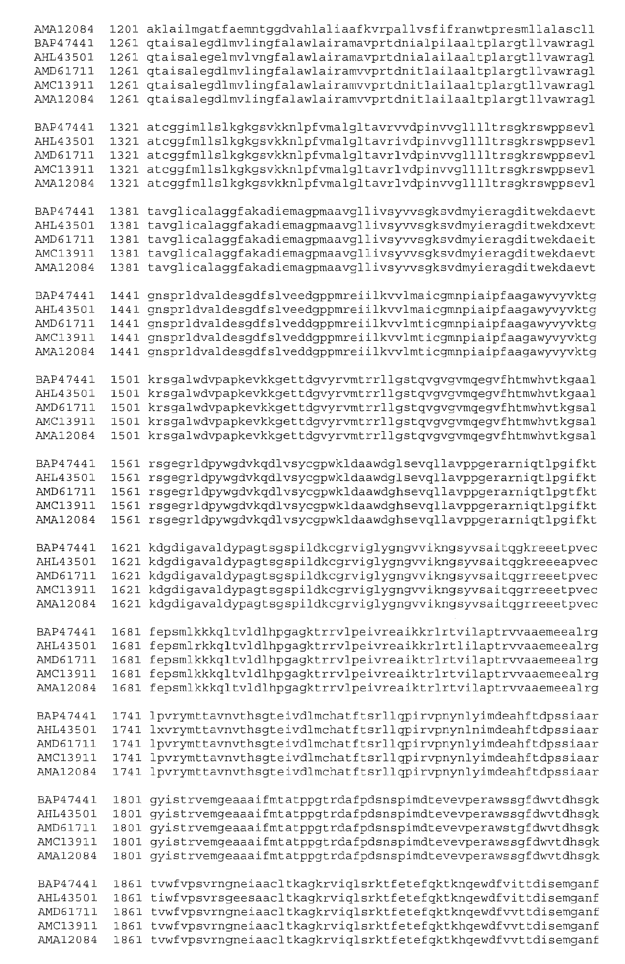

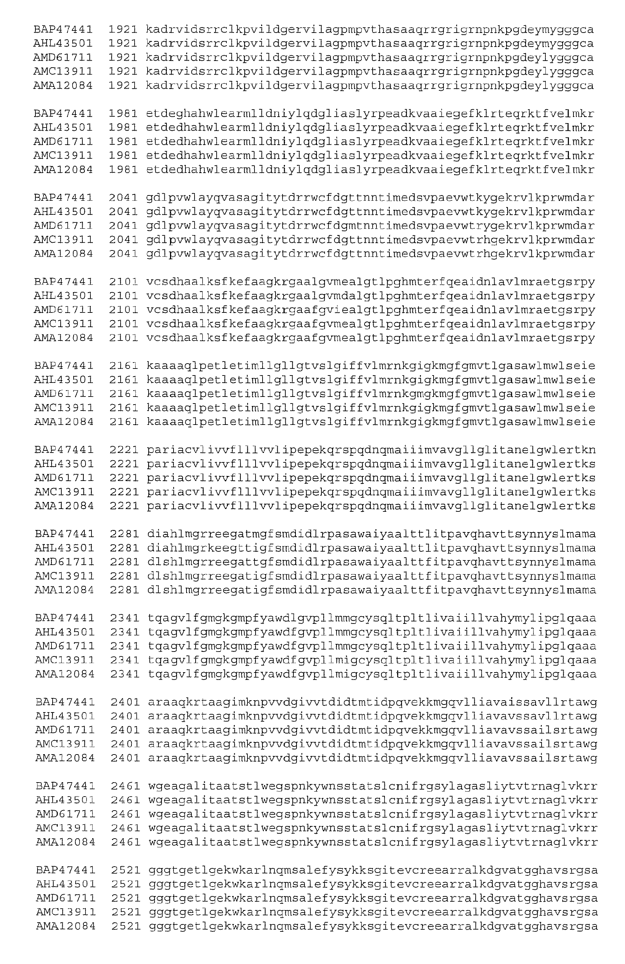

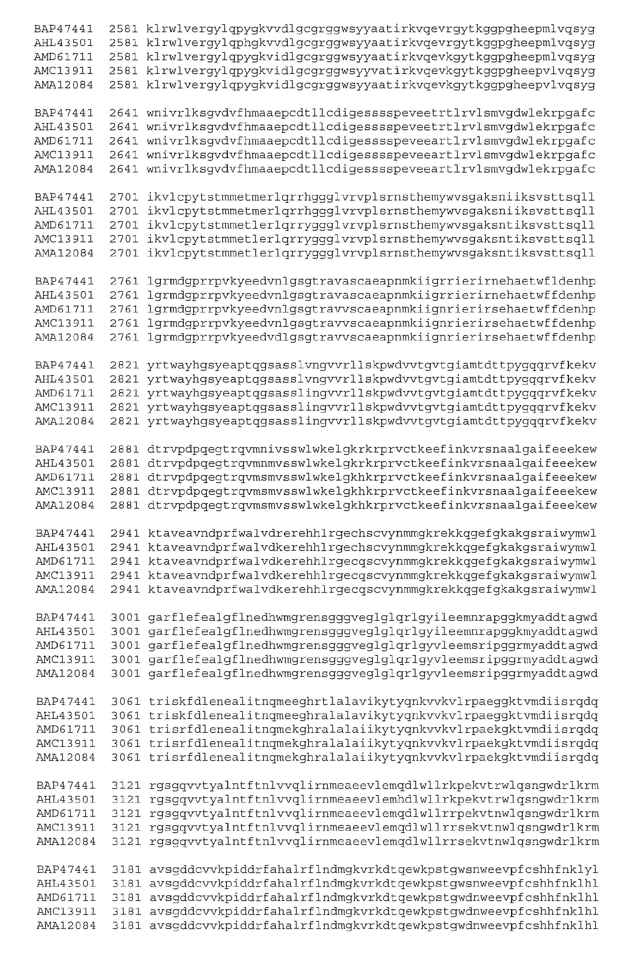

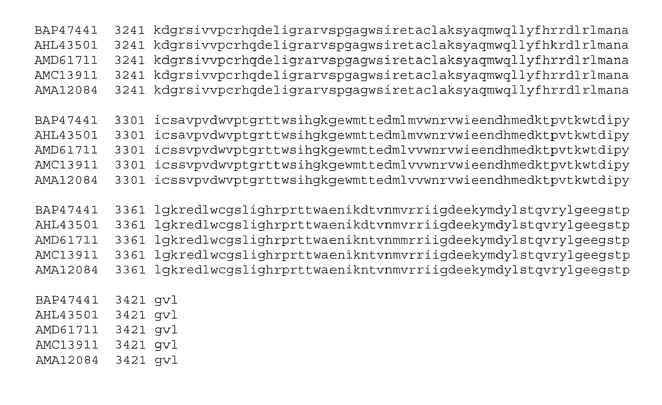

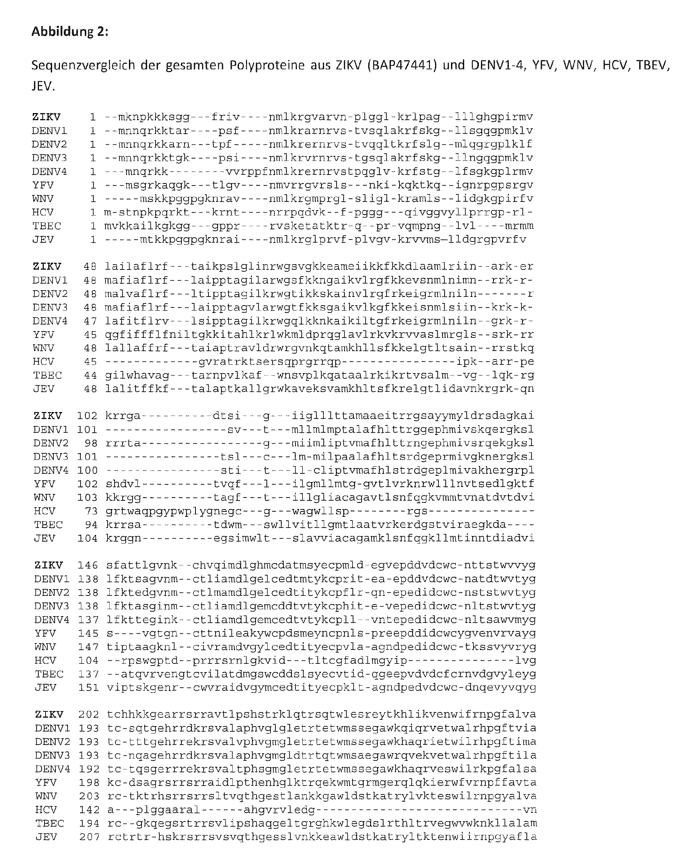

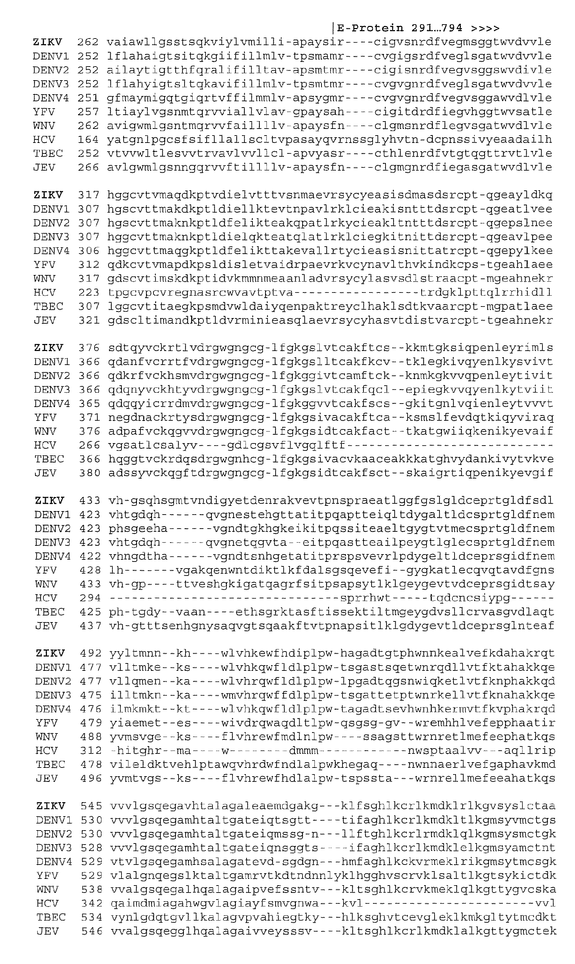

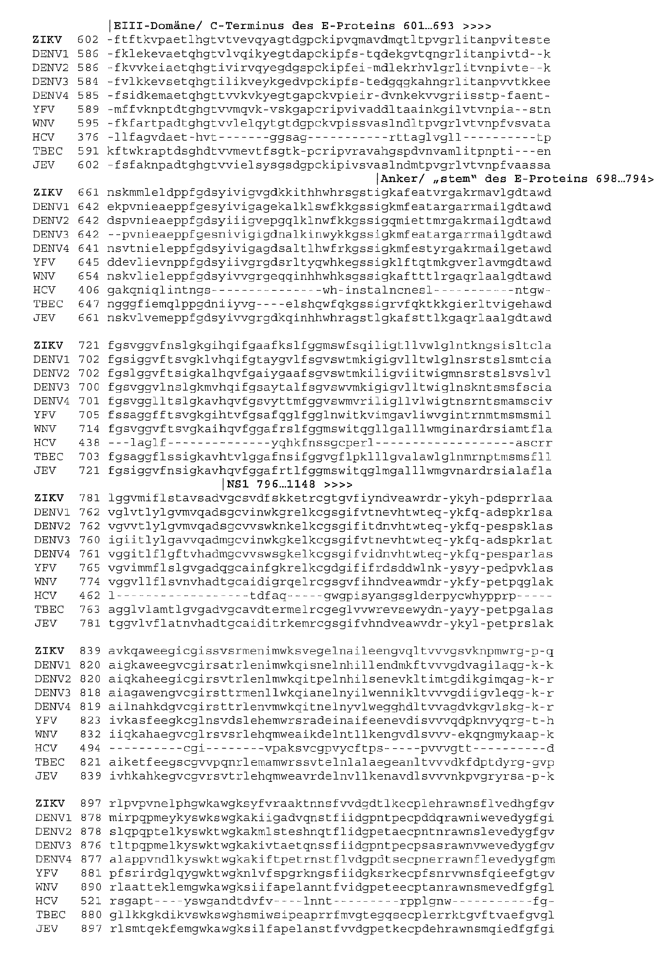

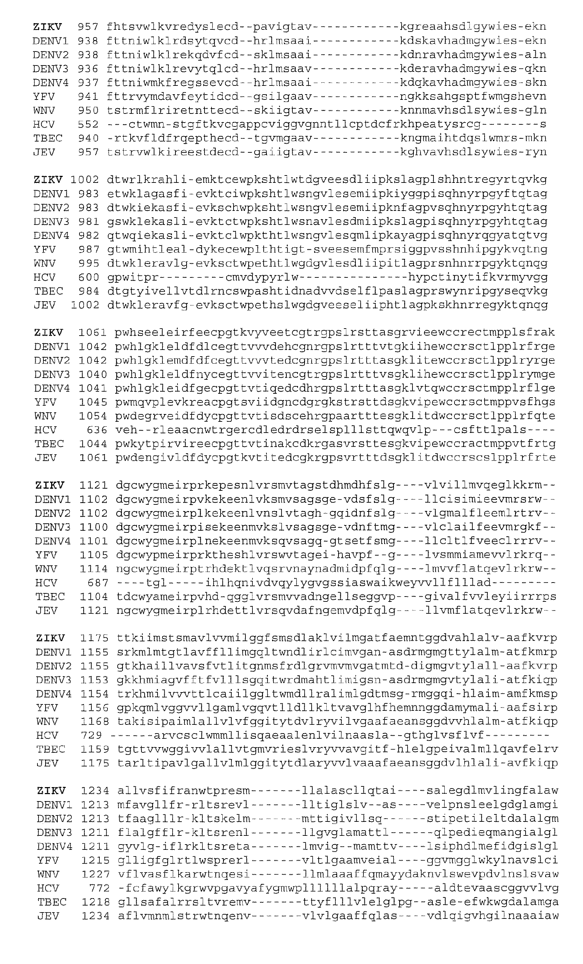

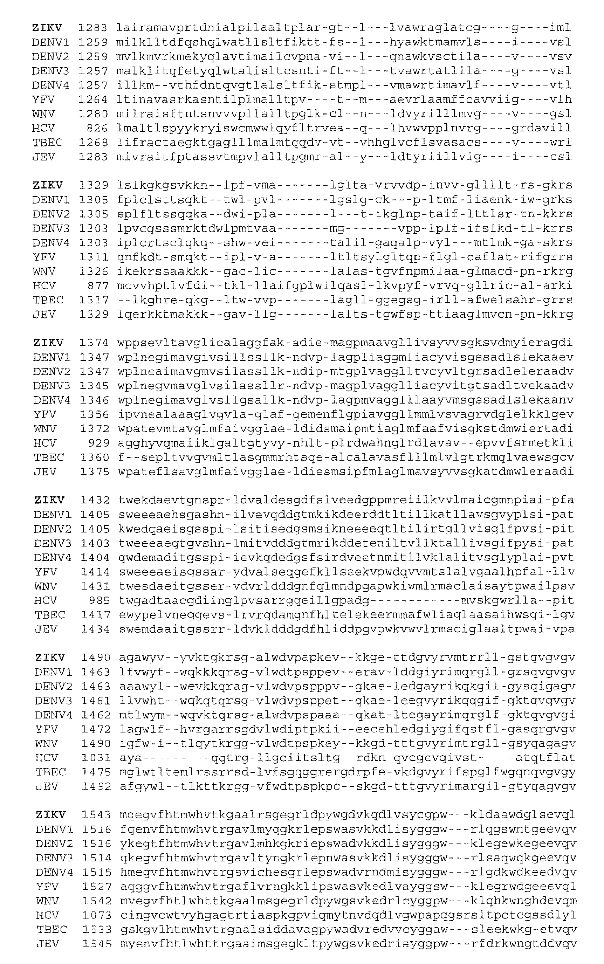

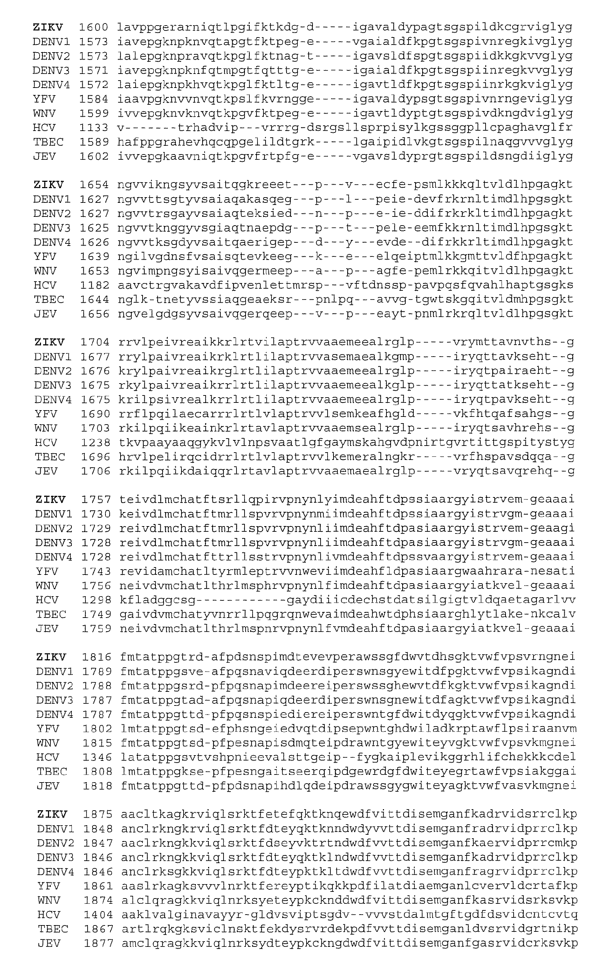

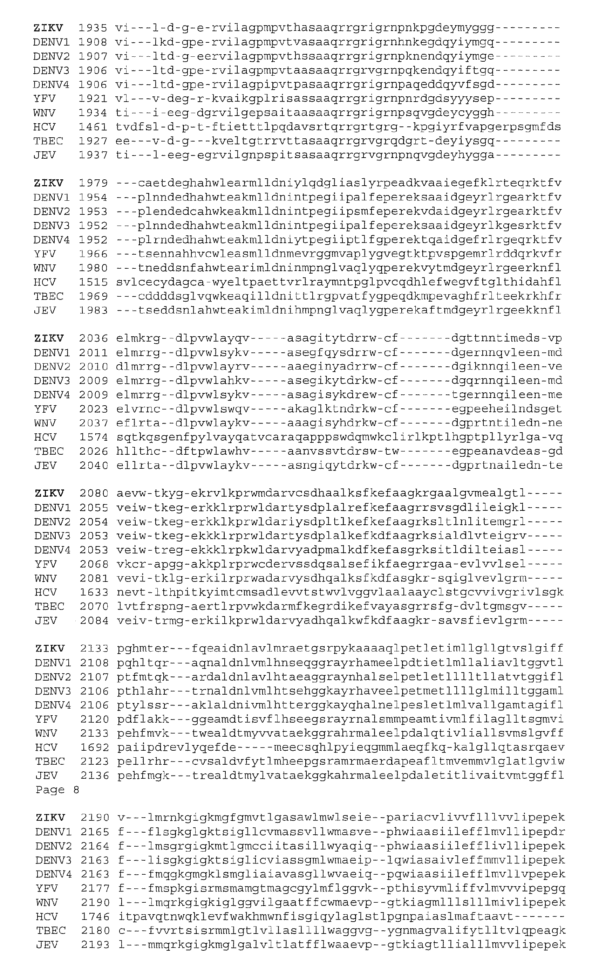

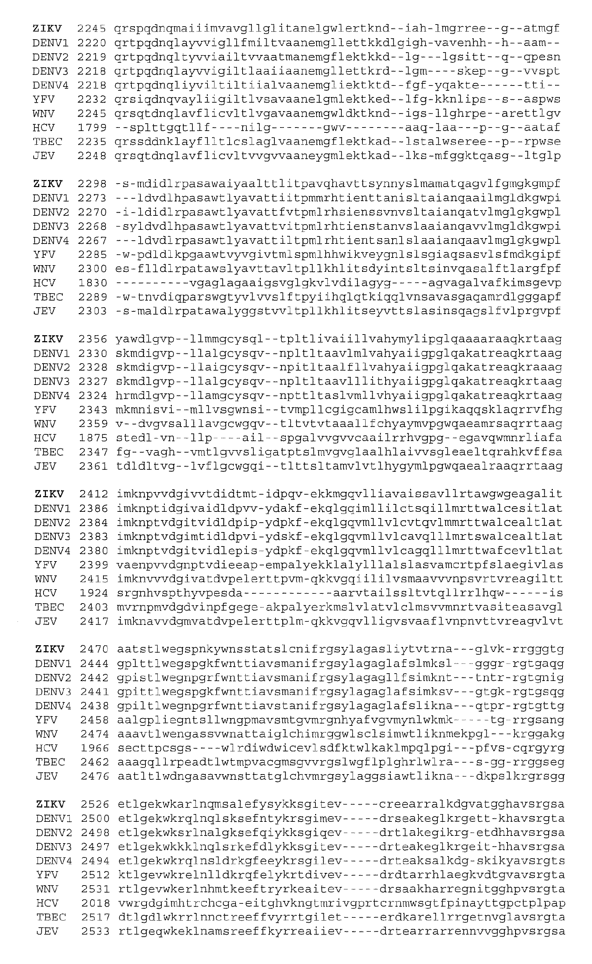

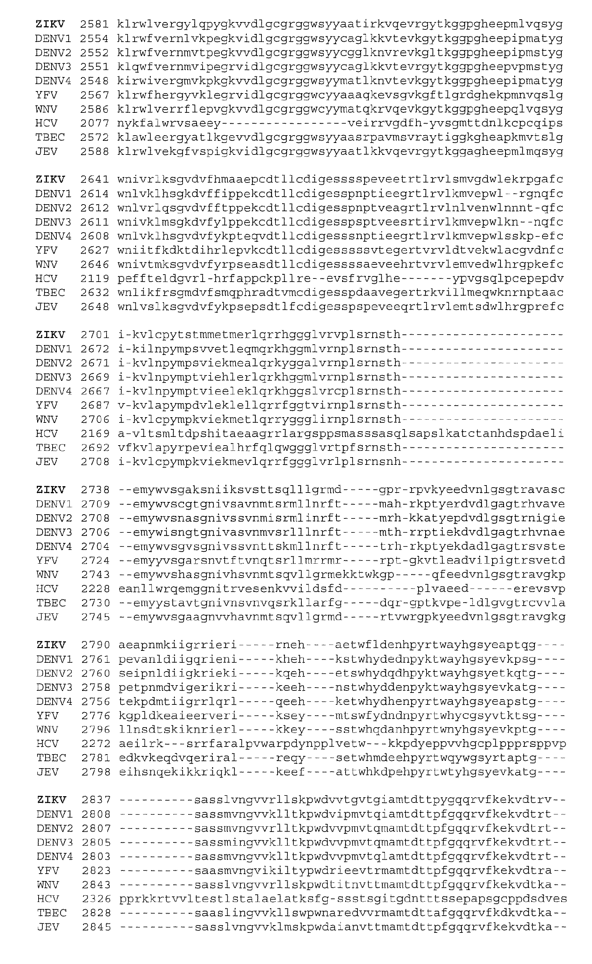

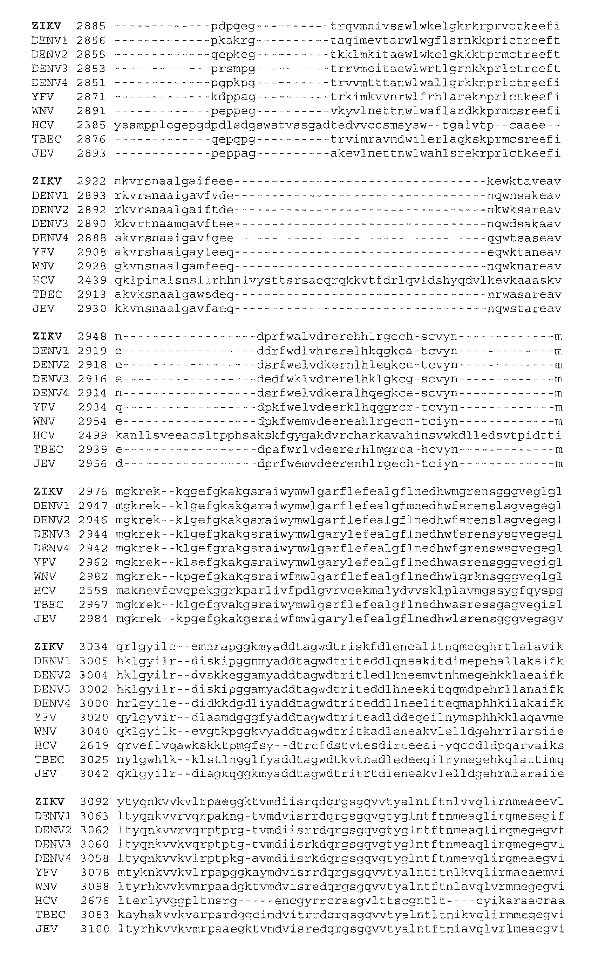

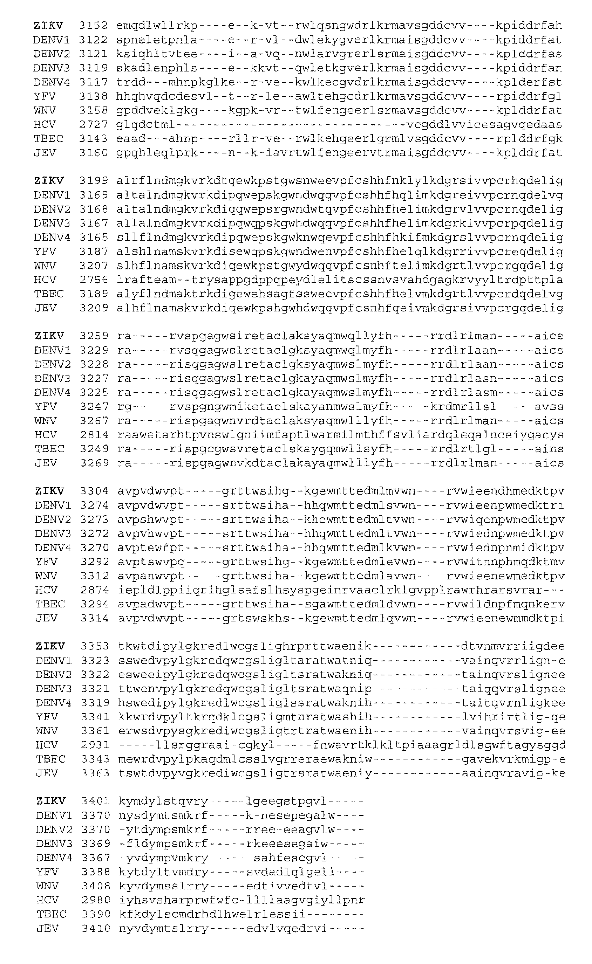

Durch Sequenzanalysen sind zahlreiche verschiedene ZIKV Stämme und deren phylogenetischer Zusammenhang bekannt. Im Wesentlichen gibt es eine afrikanische und eine asiatische Linie (Haddow et al. 2012, Faye et al., 2014), wobei die aktuellen ZIKV Isolate aus Süd- und Mittelamerika der asiatischen Linie zuzuordnen sind (Musso et al., 2015, Enfissi et al., 2016). **Abbildung 1** zeigt einen Sequenzvergleich der Polyproteine aus den folgenden, exemplarischen ZIKV Stämmen: Stamm MR766, Uganda 1947 (BAP47441), Stamm ArD7117, Senegal 1968 (AHL43501), Stamm CPC-0740, Philippinen 2012 (AMD61711), Stamm PRVABC59, Puerto Rico 2015 (AMC13911), Stamm BeH818995, Brasilien 2015 (AMA12084).

In den etwa ersten 7 Tagen einer Zika Virus Infektion kann virale RNA im Serum nachgewiesen werden, in einzelnen Fällen in Sperma sogar länger. Mittels RT-PCR kann das Virus in diesem Zeitfenster eindeutig nachgewiesen und beispielsweise von Dengue (DENV) oder Chikungunya (CHIKV) unterschieden werden. Ein negatives RT-PCR Ergebnis schließt eine Infektion jedoch bereits 5-7 Tage nach Auftreten der ersten Symptome nicht mehr aus, weil Virämie und damit RNA-Menge schnell abnehmen (Bulletin of the World Health Organization, BLT.16.171207).

Durch Nachweis des Virus über die Anwesenheit von NS1-Antigen im Serum vergrößert sich das Zeitfenster nicht wesentlich gegenüber der RT-PCR während der NS1-Antigennachweis gleichzeitig nicht die Spezifität der RT-PCR erreicht und die Gefahr von Verwechslungen mit DENV Infektionen mit sich bringt (Gyurech et al., 2016).

Nachteilig ist somit das kurze Zeitfenster für den spezifischen Nachweis des ZIKV, insbesondere weil die vergleichsweise milden klinischen Symptome, dazu führen können, dass Patienten erst spät einen Arzt aufsuchen. Nur eine von 5 Personen entwickelt überhaupt Symptome, die insbesondere als Hautausschlag und Fieber wenig spezifisch sind und meist nach wenigen Tagen abklingen. Dennoch ist die Diagnostik auch ohne klinische Symptome von allergrößter Bedeutung, weil es sowohl Hinweise auf die sexuelle Übertragbarkeit von ZIKV (Foy et al., 2011; Musso et al., 2015; McCarthy, 2016, Mansuy et al., 2016) als auch die vorallem in Südamerika auffällige Häufung von Mikrozephalie bei Neugeborenen gibt, die in Zusammenhang mit ZIKV Infektionen gebracht wird (Cauchemez et al 2015, Calvet et al 2016, Mlakar et al. 2016).

Das größte Zeitfenster kann durch serologische Teste abgedeckt werden, die insbesondere anti-Zika Virus IgM- und IgG-Antikörper im Blut (Serum oder Plasma) nachweisen.

Im Stand der Technik bekannt sind Immunfluoreszenzteste, wie beispielsweise Arboviren-Fieber-Mosaik 2 (IgG/IgM (Euroimmun AG, Deutschland) sowie IgM-Fänger-ELISA Testeallen voran MAC-ELISA (CDC, USA) mit ganzen Viruspartikeln aus infizierten Vero-Zellen. Die Hauptmenge des Zika Virus Antigens stellt in diesen Fällen das E-Protein dar, das jedoch nicht hoch-spezifisch ist und insbesondere mit Antikörpern gegen andere Flaviviren bzw. Arboviren kreuzreagiert. Besonders zu beachten sind hierbei auch Impftiter, beispielsweise gegen FSME/TBEV oder Gelbfieber.

ELISA-Teste wie beispielsweise Anti-Zika-Virus-ELISA (IgG/IgM) (Euroimmun AG, Deutschland), ZV-IgG Elisa kit Human Zika Virus IgG (ZV-IgG) (MyBioSource, USA) zum Nachweis von anti-ZIKV Antikörpern verwenden daher bevorzugt (sofern angegeben) das NS-1 Antigen, das als hochspezifisch beschrieben ist.

Zunehmend werden auch Schnellteste angeboten wie beispielsweise Zika Virus Rapid Test (Biocan Diagnostics Inc., Kanada), Zika Virus IgG/IgM RapiCard™ InstaTest (Cortez Diagnostics Inc., USA)

In allen Fällen handelt es sich um Suchteste, auf die in der serologischen Diagnostik bevorzugt ein Bestätigungstest folgt. Bestätigungsteste überprüfen zum einen die Spezifität des Suchtestergebnisses und differenzieren zum anderen das Antikörperspektrum genauer, um Rückschlüsse auf den Status der Infektion zu ermöglichen. Letzteres ist mit einzelnen ELISA-Testen in der Regel schwer oder überhaupt nicht möglich, da diese nur einen einzigen Messwert pro Probe liefern.

Aus diesem Grund handelt es sich bei Bestätigungstesten oft um Multiplex-Teste. Unter erfindungsgemäßen Multiplex Testen werden solche Einrichtungen und Verfahren verstanden, die gleichzeitig mehr als einen Messwert pro Probe liefern. ZIKV Bestätigungsteste sind jedoch weder kommerziell verfügbar, noch beschrieben.

Aufgabe der vorliegenden Erfindung ist die Bereitstellung von Mitteln und Verfahren zur Diagnostik von ZIKV Infektionen, insbesondere -aber nicht beschränkt auf-serologische Bestätigungsteste zum Nachweis von anti-ZIKV Antikörpern.

Ein erfindungsgemäßer ZIKV Bestätigungstest kann beispielsweise als Träger oder Testkit realisiert werden, enthaltend (jedoch nicht beschränkt auf): verschiedene ZIKV Antigene, verschiedene Konzentrationen desselben ZIKV Antigens zur Titerbestimmung bzw. Reaktivitäts- oder Aktivitätsbestimmung, zusätzliche Immunglobulinklassen-spezifische Fängerantikörper z.B. für sogenannte "MAC"- bzw. "µ-capture" Teste, die nur IgM Antikörper aus dem Serum binden, zusätzliche Fängerantikörper zum parallelen ZIKV-Antigen-Nachweis (Combo-Assay), Antigene potentiell kreuzreaktiver Pathogene/ entsprechender Impfstoffe (DENV, WNV, YFV, TBEC, SLV, JEV, CHIKV) zur Differentialdiagnostik.

Bei der Entwicklung eines erfindungsgemäßen Trägers oder Testkits ist neben der Auswahl der Antigene, die eine hohe Sensitivität und Spezifität aufweisen, insbesondere die geeignete Kombinatorik von Bedeutung. Im Fall von bekannten Kreuzreaktionen soll der erfindungsgemäße Bewertungsalgorithmus für einen Multiplextest bevorzugt die Sensitivität verschiedener Antigene ausschöpfen und gleichzeitig einzelne Kreuzreaktivitäten bzw. Unspezifitäten eines Antigens durch mindestens ein anderes Antigen ausschliessen.

Im einfachsten Fall wird ein 2-Antigen-Kriterium verwendet, beispielsweise mit den Antigenen E und NS1. Sind zum Beispiel Kreuzreaktivitäten bei anderen Infektionen bekannt, können diese explizit durch ein oder mehrere für diese andere Infektion spezifische(s) Antigen(e) ausgeschlossen werden. Zu solchen Antigenen zur Ausschluss-Diagnostik zählen - jedoch nicht darauf begrenzt - Struktur- und Nicht-Strukturproteine anderer Flaviviren wie DENV, WNV, YFV, TBEC, SLV, JEV, aber auch entsprechende Proteine anderer differentialdiagnostisch relevanter Infektionen mit Arboviren wie CHIKV bis hin zum Epstein-Barr Virus (EBV).

In einer Ausführungsform kann der erfindungsgemäße Diagnostik Test zusätzlich mindestens ein Antigen aufweisen, das in Suchtesten (Screening) verwendet wird. Beispiele für ein solches Such-(Screening-) Antigen sind gereinigte ZIKV Viruspartikel aus infizierten Vero-Zellen oder anderer Zelllinien bzw. entsprechendes Lysat solcher Viruspartikel. Auf diese Weise kann das Suchergebnis überprüft werden. Es ist aber ebenso möglich, Such- und Bestätigungstest in einem Schritt durchzuführen und den Multiplextest bereits als Eingangstest einzusetzen.

Gegenstand der Erfindung sind Träger zum Nachweis von anti-ZIKV Antikörpern, umfassend mindestens zwei von dem Zika Virus abgeleitete Antigene, welche an anti-ZIKV Antikörper binden können und getrennt voneinander detektierbar sind, wobei die Proteine unabhängig voneinander auf den Träger beladen werden.

Prinzipiell sind alle Materialien zum Aufbau des erfindungsgemäßen Trägers geeignet, die dem Fachmann zur Immobilisierung von Antigenen bekannt sind. Hierzu zählen Platten, Streifen, Membranen, Filter, Papier, Film oder Perlen. Als Platten kommen insbesondere Mikrotiterplatten in Frage. Die Perlen können auch Kügelchen aus verschiedenem Material sein, an welchem die Antigene aufgetragen oder immobilisiert sind.

In einer besonders bevorzugten Ausführungsform besteht der Träger jedoch aus Material ausgewählt aus der Gruppe von Nitrocellulose, Polyvinylidendifluorid (PVDF), Polyamid, Celluloseacetat und Polystyrol. Für den Fall, dass Polyamid als Material verwendet wird, ist Nylon die bevorzugte Wahl. Nitrocellulose ist jedoch aufgrund ihrer physikalischen Oberflächeneigenschaften das zur Immobilisierung der Antigene vor allen zu bevorzugende Material. Insbesondere hat sich gezeigt, dass beim Einsatz von Nitrocellulose ein besonders gutes Signal-Rauschverhältnis für alle diagnostisch relevanten Antigensignale erreicht werden kann.

Der Träger kann weiterhin ein beschichteter Träger sein. Dann besteht der beschichtete Teil des Trägers vorzugsweise aus einem festen Material mit solchen Eigenschaften, die für eine Beschichtung geeignet ist. Diese sind dem Fachmann bekannt. Die Beschichtung des Trägers umfasst dabei vorzugsweise ein Material ausgewählt aus der obigen Gruppe von erwähnten Materialien. In einer alternativen Ausführungsform umfasst die Beschichtung des Trägers ein Material, dass zur spezifischen Bindung definierter Bindungspartner geeignet und dem Fachmann ebenfalls bekannt ist, beispielsweise Streptavidin. Vorzugsweise sind der beschichtete Teil und die Beschichtung so miteinander verbunden, dass ein unbeabsichtigtes Lösen beider Teile nicht möglich ist.

In einer alternativen Ausführungsform kann jedoch auch ein beschichteter Träger verwendet werden, dessen Beschichtung gerade ablösbar mit dem beschichteten Teil verbunden ist, wenn die Beschichtung beispielsweise in einem standardisierten Verfahren weiter zur Analyse dienen soll.

Die Antigene E, EIII, EIII* und NS-1 sind in Tabelle 1 gezeigt.

Die verwendeten Antigene können erfindungsgemäß unmittelbar auf den beschichteten Träger aufgetragen werden oder zusammen mit dem Beschichtungsmaterial auf den zu beschichtenden Teil aufgetragen werden.

In einer bevorzugten Ausführungsform ist der Träger mit Hilfe einer Technik hergestellt, welche die aufgereinigten Antigene auf bestimmte Positionen aufträgt. Die Position kodiert somit das Antigen und gewährleistet damit die genaue Zuordnung aller Antigene (Positionsmultiplexing). Bevorzugt wird die Positionszuordnung durch geeignete Computerprogramme/ Software unterstützt.

In einer alternativen Ausführungsform des Multiplexing ist der Träger eine Mischung verschiedener Perlen oder Kügelchen, mit denen die verwendeten Antigene verbunden sind, beispielsweise durch kovalente Kopplung oder passive Adsorption. Hierbei gibt es verschiedene Arten von Kügelchen, die voneinander unterscheidbar sind. Jeweils eine Art von Kügelchen wird mit einem Antigen gekoppelt, sodass die Art des Kügelchens das Antigen kodiert. Unterscheidungsmerkmale der Kügelchen sind dem Fachmann bekannt, beispielsweise Größe, magnetische Eigenschaften oder Fluoreszenz-Signal, letzteres insbesondere als Verhältnis mehrerer unterschiedlicher Fluoreszenz-Farben nebeneinander.

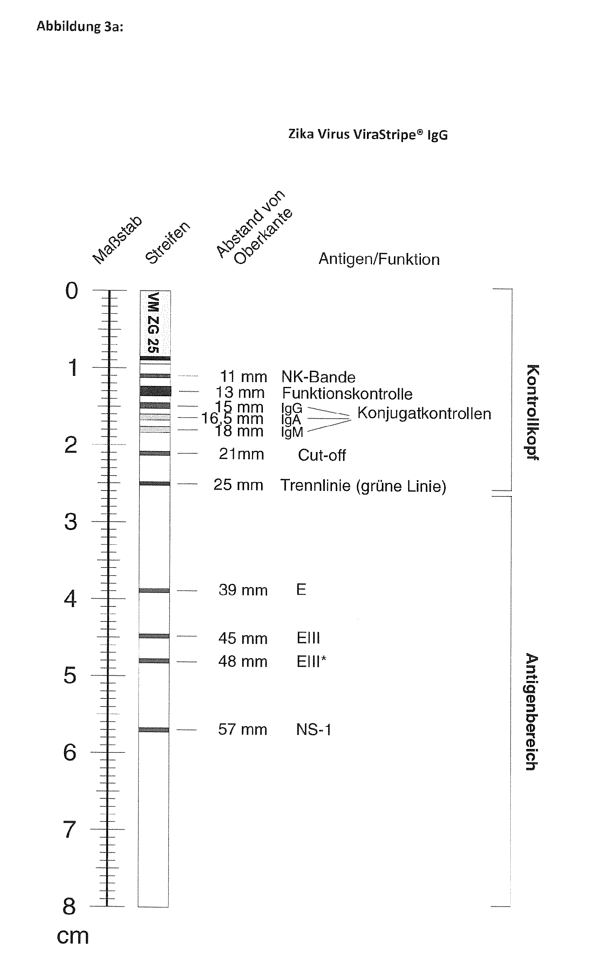

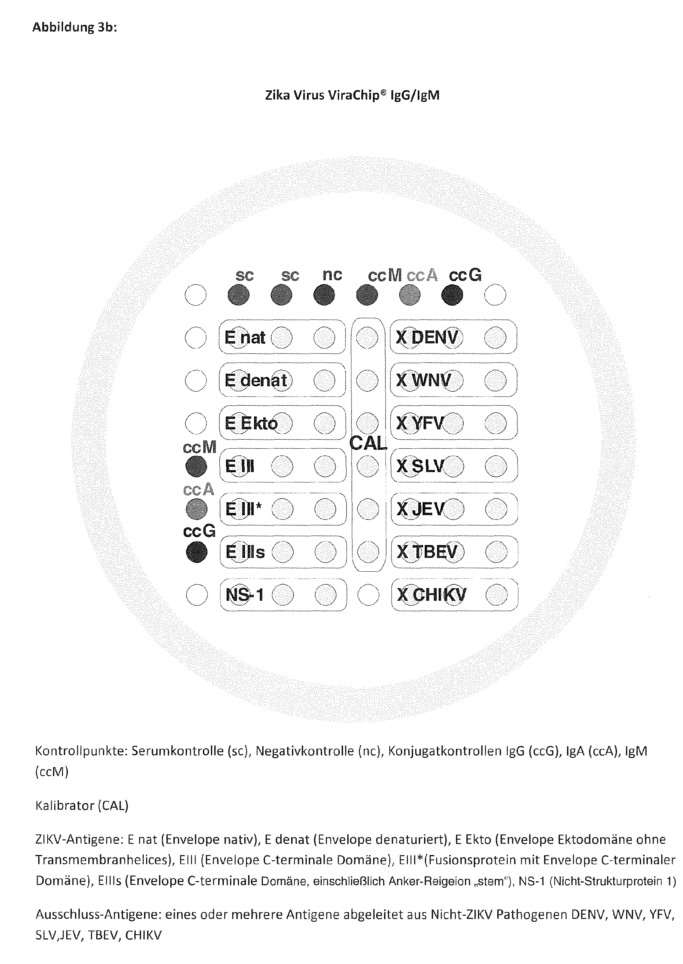

Beispielhafte, in keiner Weise limitierende Ausführungsformen sind in Abbildung 3 dargestellt. Abbildung 3a zeigt einen Nitrocellulose-Teststreifen, beladen mit 4 ZIKV-Antigenen E, EIII, EIII*, NS-1 (Zika Virus ViraStripe^{®}). Abbildung 3b zeigt ein Protein-Microarray im Mikrotiterplatten (MTP) Format (ViraChip^{®}), das neben denselben 4 ZIKV-Antigenen E, EIII, EIII*, NS1 sowohl weitere ZIKV-Antigene einschließlich Viruslysat, als auch differentialdiagnostisch relevante Antigene anderer Pathogene wie DENV, WNV, YFV, CHIKV enthält.

**Tabelle 1: Aminosäuresequenzen der erfindungsgemäßen ZIKV-Antigene E, EIII, EIII*, NS-1**

| **SEC_ID_NR** | **Protein** | **Sequenz** |
|---|---|---|
| SEQ**_**ID**_**NR1 | E | IRCIGVSNRDFVEGMSGGTWVDVVLEHGGCVTVMAQDKPTVDIELVTTTVSNMAEVRSYC YEASISDMASDSRCPTQGEAYLDKQSDTQYVCKRTLVDRGWGNGCGLFGKGSLVTCAKFT CSKKMTGKSIQPENLEYRIMLSVHGSQHSGMTVNDIGYETDENRAKVEVTPNSPRAEATL GGFGSLGLDCEPRTGLDFSDLYYLTMNNKHWLVHKEWFHDIPLPWHAGADTGTPHWNNKE ALVEFKDAHAKRQTVVVLGSQEGAVHTALAGALEAEMDGAKGKLFSGHLKCRLKMDKLRL KGVSYSLCTAAFTFTKVPAETLHGTVTVEVQYAGTDGPCKIPVQMAVDMQTLTPVGRLIT ANPVITESTENSKMMLELDPPFGDSYIVIGVGDKKITHHWHRSGSTIGKAFEATVRGAKR MAVLGDTAWDFGSVGGVFNSLGKGIHQIFGAAFKSLFGGMSWFSQILIGTLLVWLGLNTK NGSISLTCLALGGVMIFLSTAVSA |
| SEQ_ID_NR2 | EIII | SLCTAAFTFTKVPAETLHGTVTVEVQYAGTDGPCKIPVQMAVDMQTLTPVGRLITANPVI TESTENSKMMLELDPPFGDSYIVIGVGDKKITHHWHRSGST |
| SEQ**_**ID**_**NR3 | EIII* | SYSLCTAAFTFTKVPAETLHGTVTVEVQYAGTDGPCKIPVQMAVDMQTLTPVGRLITANP VITESTENSKMMLELDPPFGDSYIVIGVGDKKITHHWHRSGSTIGKAFEATVRGAKRMAV LG |
| SEQ**_**ID**_**NR4 | NS-1 | VGCSVDFSKKETRCGTGVFIYNDVEAWRDRYKYHPDSPRRLAAAVKQAWEEGICGISSVS RMENIMWKSVEGELNAILEENGVQLTVVVGSVKNPMWRGPQRLPVPVNELPHGWKAWGKS YFVRAAKTNNSFVVDGDTLKECPLEHRAWNSFLVEDHGFGVFHTSVWLKVREDYSLECDP AVIGTAVKGREAAHSDLGYWIESEKNDTWRLKRAHLIEMKTCEWPKSHTLWTDGVEESDL IIPKSLAGPLSHHNTREGYRTQVKGPWHSEELEIRFEECPGTKVYVEETCGTRGPSLRST TASGRVIEEWCCRECTMPPLSFRAKDGCWYGMEIRPRKEPESNLVRSMVTAGS |

Unter *von dem Zika Virus abgeleiteten Antigenen* werden erfindungsgemäß Präparationen verstanden, die diagnostisch relevante Antigene des Zika Virus enthalten. Dabei handelt es sich vorzugsweise um Antigene mit hoher Spezifität für ZIKV und hoher Reaktivität in verschiedenen Stadien einer ZIKV-Infektion.

Besonders bevorzugt sind ZIKV-Antigene, die sowohl Antikörper gegen die im Anwendungsgebiet des Testes vorkommenden ZIKV-Stämme mit hoher Sensitivität erfassen als auch geringe Kreuzreaktivitäten mit Antikörpern gegen andere Pathogene im selben Anwendungsgebiet aufweisen, wobei diese Antikörper anderer Pathogene entweder aus natürlicher Infektion oder aus Impfung resultieren können.

Ganz besonders bevorzugt sind Teile von ZIKV-Antigenen, die die genannte hohe Sensitivität des vollständigen ZIKV-Antigens besitzen, jedoch kreuzreaktive oder gar unspezifische Anteile nicht mehr aufweisen. Solche Teile von ZIKV-Antigenen können beispielsweise durch verkürzte Fragmente von Struktur- oder Nichtstruktur-Proteinen aus dem ZIKV erhalten werden, die mit gentechnischen Methoden rekombinant hergestellt werden.

Proteine oder Teile davon können erfindungsgemäß in unterschiedlicher Form als ZIKV-Antigen eingesetzt werden. Hierzu zählen: gefaltetes Protein in nativer Konformation, denaturiertes Protein mit linearen Epitopen, rekombinantes Fusionsprotein aus einem oder mehreren verschiedenen ZIKV-Proteinen und/oder Nicht-ZIKV-Proteinen bzw. -peptiden.

Die Verwendung von erfindungsgemäßen Nicht-ZIKV-Proteinen oder -peptiden, die mit ZIKV-Proteinen oder Teilen davon fusioniert sind beinhaltet die folgenden exemplarischen Proteine bzw. Peptide, ist jedoch nicht auf diese begrenzt: Bovines Serum Albumin (BSA), Casein, Intimin, Luciferase, PeIB, OmpT, poly-Glutamat-Tag, Glutathion-S-Transferase-(GST)-Tag, Green fluorescent protein-Tag (GFP-Tag), poly-Histidin-Tag, Intein-Tag, Maltose binding protein-Tag (MBP-Tag), MHC-Tag, Profinity eXact-Tag, Streptavidin-Tag (Strep-Tag), Tandem Affinity Purification-Tag (TAP-Tag), S-Tag, Nus-Tag, Myc-Tag, Small Ubiquitin-related Modifier (SUMO), H, Fh8, Dihydrofolat-Reduktase (DHFR-Tag).

Die Fusionierung mit diesen sowie weiteren Proteinen durch gentechnische Methoden sind dem Fachmann bekannt, um kleine und/oder unlösliche und/oder strukturell unzureichend exponierte Proteinfragmente in unterschiedlichen prokaryontischen bzw. eukaryontischen Expressionssystemen, beispielsweise *E.coli,* SF9, *S. cerevisiae* oder CHO-Zellen mit guter Ausbeute exprimieren zu können. Ebenso kennt der Fachmann die Fusion mit den genannten sowie weiteren Proteinen bzw. Peptiden als Hilfsmittel zur Affinitätsreinigung (Affinitäts-Tag) und/oder Detektionsmarkierung. Weiterhin bekannt sind Möglichkeiten, diese Fusion zu einem späteren Zeitpunkt auf Proteinebene wieder zu lösen, beispielsweise durch proteolytische Spaltung, bevorzugt durch hochspezifische Proteasen wie TEV, HRV3C, Enterokinase, Thrombin, Proteinkinase A, MMP9 (Matrix Metalloprotease 9), wobei deren Erkennungssequenz zuvor gentechnisch in das Protein eingeführt wurde. Schließlich kennt der Fachmann auch zellfreie Methoden der Proteinherstellung (in-vitro Translation). Bevorzugtes ist in allen Fällen ein ZIKV-Antigen mit optimaler Präsentation der diagnostisch relevanten Epitope.

In einer bevorzugten Ausführungsform ist der Träger so gefertigt, dass die *mindestens zwei von dem Zika Virus abgeleiteten Antigene* ausgewählt sind aus: E-Protein Voll-Länge einschließlich Transmembranhelices (SEQ ID NR 1, Aminosäuren ∼ Nr. 291...794), Ektodomäne des E-Proteins ohne Transmembranhelices (entsprechende Deletionen), EIII-Protein (SEQ ID NR 2, EIII-Domäne/ C-Terminus des E-Proteins, Aminosäuren ∼ Nr. ab 601), EIIIs-Protein (SEQ ID NR 3, EIII-Domäne/ C-Terminus des E-Proteins einschließlich des gesamten Anker/ "stem" Bereiches oder Teilen des Ankers, Aminosäuren ∼ Nr. ab 601 sowie ab 698).

Die Angaben der Aminosäuren Nr. beziehen sich hierbei auf Abbildung 1. Der Fachmann versteht diese Aminosäure-Bereiche in der Primärstruktur/ linearen Sequenz des Proteins als Anhaltspunkt zum Verständnis der dreidimensionalen Tertiär- bzw. Quartärstruktur (letztere im Fall des gesamten Virus). Die exakte Anfangs- und Endaminosäure hängt hierbei vom betrachteten Zusammenhang ab. Beispielsweise werden bei Vergleich zweier Sequenzen quantitative Regeln verwendet, die entscheiden, ob eine Aminosäure als identisch betrachtet wird oder ob diese Sequenzposition relativ verschoben wird ("gap"), um insgesamt eine bessere Übereinstimmung zu erreichen. Ebenso beispielsweise werden bei Modellierung des ZIKV-E Proteins an eine atomar aufgelöste Struktur des E-Proteins anderer Flaviviren bestimmte Annahmen für eine "beste Modellierung" gemacht, die letztlich Anfang- und End-Aminosäure Nr. einer Protein-Domäne beeinflussen.

In einer besonders bevorzugten Ausführungsform ist mindestens eines *von dem Zika Virus abgeleiteten Antigene* glykosyliert. Beispiel für eine Glykosylierungsstelle des E-Proteins ist Asn154. Dem Fachmann sind Methoden zur Glykosylierung im Rahmen der Proteinexpression bekannt, beispielsweise durch eukaryontische Expressionssysteme wie SF9-Insektenzellen. Ebenso ist dem Fachmann bekannt, dass das Glykosylierungsmuster vom Expressionssystem abhängt.

In einer weiteren Ausführungsform sind die *von dem Zika Virus abgeleiteten Antigene* mit einem Affinitäts-Tag versehen und/oder mit einem Nicht-ZIKV Protein fusioniert.

In einer ganz besonders bevorzugten Ausführungsform liegt mindestens ein *von dem Zika Virus abgeleitetes Antigen* nativ gefaltet neben mindestens einem denaturierten *von dem Zika Virus abgeleiteten Antigen* vor, sodass alle Antikörper erfasst werden die gegen native, konformative Epitope gerichtet sind und ebenso alle Antikörper gegen lineare Epitope (die im nativen Zustand unter Umständen nicht zugänglich sind).

In einer weiteren besonders bevorzugten Ausführungsform liegt mindestens ein *von dem Zika Virus abgeleitetes Antigen* in verschiedenen Konzentrationen bzw. Mengen auf dem erfindungsgemäßen Träger vor und es werden Verfahren angewendet, um auf diese Weise quantitative Aussagen zur Menge bzw. Konzentration der Antikörper in der Probe zu machen.

In einer anderen Ausführungsform liegen mindestens zwei *von dem Zika Virus abgeleitete Antigene* aus verschiedenen Stämmen des ZIKV vor.

In einer alternativen Ausführungsform sind die *von dem Zika Virus abgeleiteten Antigene* Teil eines Testsystems, z.B. eines Trägers oder Kits, das Antikörper gegen eine Vielzahl von Pathogenen nachweist, von denen einer ZIKV ist. Beispielsweise ist das Protein-Microarray im MTP Format (ViraChip^{®}) nicht nur wie oben beschrieben auf einzelne DENV, WNV, YFV, CHIKV Antigene als Ausschlusskriterium begrenzt. Vielmehr können für jeden einzelnen Erreger so viele spezifische Antigene verwendet werden, dass jeweils ein erregerspezifischer Bestätigungstest resultiert. Beispielsweise können die ZIKV-Antigene zusammen mit Antigenen, die spezifisch Antikörper gegen DENV, WNV, YFV, SLV, JEV, TBEV und andere Flaviviren nachweisen können zu einem "Flavivirus ViraChip" kombiniert werden oder zusammen mit CHIKV-Antigenen zu einem "Arbovirus ViraChip". Besonders bevorzugt werden hierbei Antigene aus verschiedenen Stämmen/ Serotypen eines Pathogens verwendet, beispielsweise DENV1, DENV2, DENV3 und DENV4.

Bereitsgestellt wird auch die Verwendung der Antigene, die auf Träger auf- oder in Kits der Erfindung eingebracht werden, zum Nachweis von ZIKV. Bevorzugt unterscheidet die Verwendung zwischen ZIKV und DENV, WNV, YFV, SLV, JEV, TBEV, CHIKV.

Gegenstand der vorliegenden Erfindung ist ebenfalls ein Verfahren zum Nachweis von Anti-ZIKV Antikörpern. Dieses Verfahren umfasst erfindungsgemäss folgende Schritte: 1) Bereitstellen eines Trägers wie oben beschrieben, 2) Inkubieren einer biologischen Probe, die möglicherweise Anti-ZIKV-Antikörper enthält, mit dem Träger unter Bedingungen, die eine Ausbildung mindestens eines Antigen-Antikörper-Komplexes erlauben; und 3) Bestimmen der Anwesenheit von Anti-ZIKV-Antikörpern in der Probe durch Nachweis mindestens eines Antigen-Antikörper-Komplexes.

Das Verfahren ist dabei ebenfalls geeignet, ein Mittel zur Diagnostik und/oder Verlaufskontrolle einer ZIKV-Infektion darzustellen. Dazu ist die biologische Probe eine Patientenprobe, das heißt eine Probe die einem menschlichen oder tierischen Körper entnommen wurde oder aber eine aus einem menschlichen oder tierischen Körper entnommene, weiterverarbeitete Probe.

Sind Kontrollsubstanzen auf dem Träger enthalten, so kann mit diesen die sachgemäße Durchführung des erfindungsgemäßen Verfahrens angezeigt werden. Beispielsweise zeigt eine Kontaktkontrolle an, ob der Träger lange genug mit der biologischen Probe in Kontakt getreten ist. Eine Serumkontrolle zeigt entsprechend an, ob während des Verfahrens der Träger mit einem Serum in Kontakt getreten ist. Erfindungsgemäss lassen die Kontrollsubstanzen auch Aussagen darüber zu, ob die Inkubation der biologischen Probe mit dem Träger für eine Ausbildung mindestens einen Antikörper-Antigen-Komplexes hinreichend lange war. In diesem Falle handelt es sich bei der Kontrollsubstanz um eine Inkubationszeitkontrolle bzw. Cutoff-Kontrolle bzw. Kalibrator-Kontrolle.

Vorzugsweise ist das Nachweisverfahren für Anti-ZIKV-Antikörper so gestaltet, dass es alle Vorgaben hinsichtlich interner und externer Qualitätssicherung sowie internationaler und nationaler Normen erfüllt.

In einer bevorzugten Ausführungsform ist das Verfahren so gestaltet, dass mindestens ein Antigen spezifisch mit einem Anti-ZIKV-Antikörper reagiert.

Die biologische Probe zur Verwendung in dem Verfahren oder auf dem Träger bzw. mit dem Kit der Erfindung kann von einem Tier oder Menschen abgeleitet sein. Besonders bevorzugt ist, dass es sich um eine Probe von einem Menschen handelt.

Vorzugsweise handelt es sich bei der biologischen Probe um eine flüssige Probe. In diesem Fall kommen insbesondere Blut-, Plasma-, Serum-, Urin-, Speichel- oder Samenflüssigkeit in Frage. Besonders vorzugsweise ist die biologische Probe humanes Blut oder Serum. Die biologische Probe kann auch eine weiterverarbeitete biologische Probe sein. Vorzugsweise ist dann die Weiterverarbeitung so gestaltet, dass die diagnostisch relevanten Antikörper im Vergleich zur ursprünglichen Probe aufkonzentriert werden, oder in höherer Reinheit vorliegen. Verfahren zur Weiterverarbeitung der biologischen Probe sind dem auf diesem Gebiet tätigen Fachmann bekannt.

Erfindungsgemäss kann die biologische Probe ebenfalls eine verflüssigte Probe sein, welche aus einer Gewebeprobe oder einem Bioptat, vorzugsweise Hautbioptat, Gelenkbioptat, Synovialbioptat durch ein Weiterverarbeitungsverfahren gewonnen wurde.

Zur Detektion eines Antigen-Antikörper-Komplexes wird in der Diagnostik von ZIKV-Infektionen der erfindungsgemässe Testkit verwendet. Ein solcher Testkit enthält neben dem Träger auch mindestens eine Substanz zum Nachweis eines Antigen-Antikörper-Komplexes und/oder mindestens eine Vorrichtung zum Nachweis eines Antigen-Antikörper-Komplexes.

Im Rahmen einer Stufendiagnostik ist es möglich, nach einem vorgeschalteten Suchtest (z. B. ELISA, Immunfluoreszenztest) mit dem erfindungsgemässen Träger, Verfahren und Testkit sowohl die serologische Bestätigungsreaktion, als auch die serologische Verlaufskontrolle durchzuführen. Die Testdaten liefern einen Teilaspekt für die Entscheidung der Behandlungsbedürftigkeit durch den behandelnden Arzt.

In einer bevorzugten Ausführungsform wird zum erfindungsgemäßen Träger oder Kit weiterhin eine Anweisung zur Auswertung der Testergebnisse bereitgestellt, welche mit dem Träger bzw. Kit gewonnen werden. Diese Anweisung soll es dem Anwender ermöglichen, Aussagen über die Anwesenheit von Anti-ZIKV-Antikörpern bzw. kreuzreagierenden Antikörpern in der biologischen Probe zu treffen. Die Anweisung ist vorzugsweise so gestaltet, dass Regeln oder Kriterien vorgegeben sind, die eine Aussage darüber ermöglichen, ob die Anwesenheit von Anti-ZIKV-Antikörpern 1) positiv ist, falls Anti-ZIKV-Antikörpern anwesend sind, oder 2) negativ ist, falls Anti-ZIKV-Antikörper im Wesentlichen nicht anwesend sind, oder 3) das Ergebnis fraglich ist, falls keine Aussage über die Anwesenheit von Anti-ZIKV-Antikörpern getroffen werden kann, oder 4) das Ergebnis fraglich ist, falls die Anwesenheit von kreuzreaktiven Antikörpern wahrscheinlich ist.

In einer anderen Ausführungsform enthält die Anweisung entsprechend Regeln und Kriterien, wie Aussagen über Konzentrationen von Anti-ZIKV-Antikörpern in der biologischen Probe gewonnen werden können.

In wieder einer anderen Ausführungsform ermöglichen Träger bzw. Kit zusammen mit der Anweisung zusätzliche Aussagen zu treffen, welche Art von Antikörpern, vorzugsweise klassifiziert nach IgG-, IgM- oder IgA-Antikörpern in der biologischen Probe vorliegen. Besonders bevorzugt lässt die Anweisung auch Aussagen darüber zu, in welchen Konzentrationen die verschiedenen Antikörper vorliegen.

In einer weiteren Ausführungsform enthält die Anweisung Informationen darüber, wie der Träger bzw. das Testkit zur Ermittlung von Aussagen verwendet werden kann, ob eine Testperson, von der die zu untersuchende biologische Probe entnommen wurde, mit ZIKV infiziert ist.

In noch einer alternativen Ausführungsform enthält die Anweisung Informationen, wie der Träger bzw. das Testkit zur Verlaufskontrolle einer ZIKV-Infektion eingesetzt werden kann. Insbesondere enthält die Anweisung dann Regeln oder Kriterien darüber, wie die Daten zu dem Krankheitsbild korreliert werden können. Die Aussage basiert dann erfindungsgemäss darauf, über das Vorhandensein von jeweils IgG-, IgM- und/oder IgA-Antikörpem und falls vorhanden, vorzugsweise auch die Konzentrationen der jeweiligen Antikörper, Rückschlüsse auf das Stadium zu treffen, in welchem sich eine Testperson mit ZIKV-Infektion befindet.

Ebenfalls kann die Anweisung Informationen darüber enthalten, wie der Testkit über einen längeren Zeitraum zur Verfolgung einer ZIKV-Infektion angewendet werden kann.

Die nachfolgenden Ausführungsbeispiele sollen die vorliegende Erfindung näher erläutern, ohne jedoch limitierend zu wirken.

Erfindungsgemäß umfasst sind nicht nur die jeweiligen Antigene, sondern auch Varianten davon.

Varianten sind solche Antigene, die bei Homologievergleich mit einer der erfindungsgemäßen Sequenzen SEQ ID NR 1 bis SEQ ID NR 4 eine Sequenzidentität mindestens 80%, bevorzugt 85%, besonders bevorzugt 90%, am meisten bevorzugt 95% aufweisen und/oder bei Verwendung unter den in Beispiel 2 angegebenen Bedingungen die gleiche Reaktivität mit einem ZIKV-Serumkollektiv von mindestens 10 verschiedenen Proben zeigen wie die erfindungsgemäßen ZIKV Antigene.

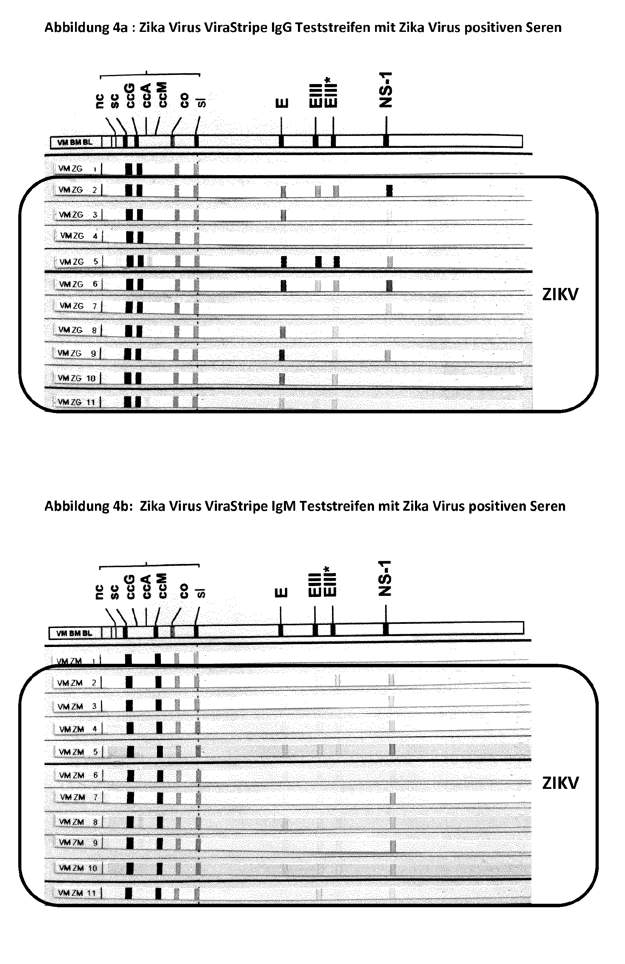

Ein ZIKV-Serumkollektiv umfasst Seren unterschiedlicher Reaktivität, beispielsweise wie in Abbildung 4 dargestellt.

Die Interpretationskriterien eines bevorzugten Trägers bzw. Testkits der Erfindung sind in Beispiel 4 dargestellt.

### Beispiele

### Beispiel 1:

### Herstellung von ZIKV-Antigenen

Die Herstellung der verschiedenen ZIKV-Antigen-Konstrukte erfolgt abgeleitet von den entsprechenden Genabschnitten des viralen RNA-Genoms. Diese werden in entsprechende Expressionsplasmide eingefügt. Es werden Techniken verwendet, die dem auf diesem Gebiet tätigen Fachmann bekannt sind.

Die Expression des Antigens EDIII erfolgt beispielsweise aus dem Vektor pET14b in E.coli BL21. Zunächst wird eine Übernachtkultur in LB-Medium angeimpft und das Antibiotikum Ampicillin zugeben. Die Inkubation erfolgt bei 37°C über Nacht im Schüttelinkubator. Als Übertagkultur wird LB-Medium mit Ampicillin mit einer oD600 von 0,04 angeimpft und bei 37°C im Schüttelinkubator inkubiert. Ist eine oD600 von ca. 0,6 erreicht, wird die Proteinexpression durch IPTG-Zugabe induziert. Die Proteinexpression erfolgt für ca. 4h bei 37°C im Schüttelinkubator.

Die E.coli Zellen werden abzentrifugiert und mittels eines Lysozym, DTT, Triton X-100 und Proteaseinhibitor enthaltenden Lysispuffers resuspendiert. Die Zellen werden mittels Ultraschall auf Eis aufgeschlossen und anschließend erfolgt ein DNase-Verdau. Das Lysats wird abzentrifugiert und das Pellet mit den unlöslichen Proteinbestandteilen noch zweimal mit einem DTT und Proteaseinhibitor enthaltendem Waschpuffer gewaschen. Anschließend wird das Pellet in einem 6M Guanidiumhydrochlorid enthaltendem Puffer aufgelöst und die unlöslichen Bestandteile mittels Zentrifugation abgetrennt.

Die Aufreinigung des Antigens EDIII erfolgt dann beispielsweise mittels Affinitätschomatographie über His-Tag unter Verwendung einer Nickel-NTA-Säule. Die Aufreinigung erfolgt denaturierend unter Verwendung von 6M Guanidiumhydrochlorid enthaltenden Puffern. Die Elution erfolgt unter Anwendung eines Immidazolgradienten und die Gipfelfraktionen werden vereinigt. Zur Umpufferung des Antigens wird beispielsweise die Zentrikon-Technologie angewendet, die dem auf diesem Gebiet tätigen Fachmann bekannt ist. Nun kann eine Abspaltung des His-Tags mittels Protease erfolgen. Als zweiten Reinigungsschritt kann zum Beispiel eine Gelfiltration durchgeführt werden mittels derer eine Auftrennung nach Größe erfolgt und weitere unspezifische E.coli Proteine sowie die Protease und der His-Tag abgereinigt werden.

### Beispiel 2:

### Beladung eines Nitrocellulose Trägers mit ZIKV-Antigenen in Linienform / Zika Virus ViraStripe^{®}

Die ZIKV-Antigen-Lösungen enthalten Konzentrationen zwischen 5 µg/ml und 50 µg/ml der erfindungsgemäßen ZIKV-Antigene und werden über einen Stripe-Prozess, der für die ViraStripe^{®} Produktlinie seit langer Zeit etabliert ist mit einem Volumen von 1 µl pro cm auf den Träger Nitrozellulose (GE Whatman, Dassel, Deutschland) aufgebracht. Hierfür wurden Dispensierapparate der Firma Biodot Ltd. (Chichester, Großbritannien) nach Angaben des Herstellers verwendet. Die Bedingungen wurden so gewählt, dass die Antigen-Lösungen als homogene Linie an definierter Position auf den Träger aufgebracht wurden (siehe Abbildung 3a). Während des Stripe-Prozesses betrug die relative Luftfeuchtigkeit 40 - 60 % und die Temperatur 19-23 deg. C. Anschließend wurden die Membranen standardmässig nach Angaben des Herstellers geblockt. Zu Bedingungen und weiterer Verwendung des Trägers siehe auch EP1726960B1, EP1556697B1.

### Beispiel 3:

### Beladung eines Trägers mit ZIKV-Antigenen sowie weiteren Antigenen in Punktform / Zika Virus ViraChip^{®}

Analog Beispiel 2 können die ZIKV-Antigen-Lösungen über einen Spotting-Prozess, der für die ViraChip^{®} Produktlinie etabliert ist auf den Träger Nitrozellulose (GE Whatman, Dassel, Deutschland) aufgebracht werden, der sich auf dem Boden eines Mikrotiterplatten (MTP) Napfes befindet. In diesem Fall werden die Antigen-Lösungen als homogene Punkte an definierter Position auf den Träger aufgebracht (siehe Abbildung 3b). Im Gegensatz zum ViraStripe^{®} Format werden Antigene im ViraChip^{®} Format typischerweise mehrfach, in Form identischer Replikate aufgetragen, um die Messwertgenauigkeit statistisch zu verbessern. Auch Kontrollpunkte liegen meist als Replikate vor. Der wichtige Kalibrator (CAL) wird sogar sechsfach aufgetragen.

### Beispiel 4:

### Interpretationskriterien für ein Testergebnis mit den ZIKV-Antigenen E, EIII, EIII* und NS1

### IgG Interpretationskriterien

Allgemein gilt: Die Bandenintensitäten werden anhand der Cut off Kontrolle bestimmt. Diese befindet sich im Kontrollabschnitt auf jedem Teststreifen. Deutliche Banden haben eine Intensität ≥ der Cut off Kontrolle. Schwache Banden haben eine Intensität < der Cut off Kontrolle.

| **Auftretende Banden** | **Bewertung / Ergebnis** | **Beurteilung** |
|---|---|---|
| Eine **deutliche NS1** Bande und mindestens eine **schwache** Bande aus **E, EIII, EIII*.** | **Positiv** | Spezifische IgG Antikörper gegen **Zika Virus** nachweisbar. Eine Infektion mit dem **Zika Virus** ist wahrscheinlich. |
| Eine **schwache NS1** Bande und eine **deutliche E** Bande. | | |
| Eine **deutliche NS1** Bande singulär oder eine **deutliche E** Bande und eine **deutliche EIII oder EIII*** Bande | **Grenzwertig** | Spezifische IgG Antikörper gegen **Zika Virus** nachweisbar. Eine Infektion mit dem **Zika Virus** ist möglich. Bei Verdacht auf IgM spezifische Antikörper prüfen und mit einer zweiten Serumprobe für IgG and IgM nach 2-3 Wochen überprüfen. |
| Keine Banden oder alle anderen Kombinationen | **Negativ** | Keine spezifische IgG Antikörper gegen **Zika Virus** nachweisbar. Bei Verdacht auf IgM spezifische Antikörper prüfen und mit einer zweiten Serumprobe für IgG and IgM nach 2-3 Wochen überprüfen. |

### IgM Interpretationskriterien

Allgemein gilt: Die Bandenintensitäten werden anhand der Cut off Kontrolle bestimmt. Diese befindet sich im Kontrollabschnitt auf jedem Teststreifen. Deutliche Banden haben eine Intensität ≥ der Cut off Kontrolle. Schwache Banden haben eine Intensität < der Cut off Kontrolle.

| **Auftretende Banden** | **Bewertung / Ergebnis** | **Beurteilung** |
|---|---|---|
| Eine **deutliche NS1** Bande | **Positiv** | Spezifische IgM Antikörper gegen **Zika Virus** nachweisbar. Eine Infektion mit dem **Zika Virus** ist wahrscheinlich. |
| Eine **schwache NS1** Bande oder zwei **schwache Banden** aus **E, EIII, EIII*** | **Grenzwertig** | Spezifische IgM Antikörper gegen **Zika Virus** nachweisbar. Eine Infektion mit dem **Zika Virus** ist möglich. |
| **Keine** Banden oder alle anderen Kombinationen | **Negativ** | Keine spezifischen IgM Antikörper gegen **Zika Virus** nachweisbar. Bei Verdacht auf IgM spezifische Antikörper prüfen und mit einer zweiten Serumprobe für IgG and IgM nach 2-3 Wochen überprüfen. |

### Beispiel 5: Reaktivität des erfindungsgemäßen Nitrocellulose-Trägers mit den ZIKV-Antigenen E, EIII, EIII* und NS1 in Linienform (Zika Virus ViraStripe^{®} IgG/IgM) mit ZIKV-Seren bzw. DENV, WNV und TBEC Seren

In einem Test mit 10 ZIKV Seren, die durch Immunfloureszenztests gesichert IgG und IgM Antikörper gegen ZIKV aufweisen sowie im entsprechenden Virusneutralistionstest positiv reagierten, wurden Reaktivitäten mit dem Zika Virus ViraStirpe^{®} IgG und IgM bestimmt (siehe Abbildung 4).

Überraschenderweise zeigen Fragmente des Envelope (E) - Proteins bei Verwendung als ZIKV-Antigene in geeigneter Konzentration auf dem erfindungsgemäßen Träger in manchen Fällen eine höhere Reaktivität mit ZIKV positiven Seren als das gesamte E-Protein, möglicherweise aufgrund von besserer Exposition relevanter Epitope auf dem Träger. Besonders in einem Fall konnten nur Antikörper gegen die Fragmente des Envelope (E) - Proteins gefunden werden, hingegen keine Antikörper gegen das vollständige E Protein oder NS1. Ein positives Ergebnis konnte somit nur durch den Nachweis von Antikörpern gegen die Fragmente des Envelope (E) - Proteins erhalten werden. Bei dieser Probe zeigte sich weiterhin, dass die zugehörigen Titerbestimmungen aus der Immunfluoreszenz niedrig waren und somit auf eine möglicherweise noch frühe Phase der Erkrankung hinweisen.

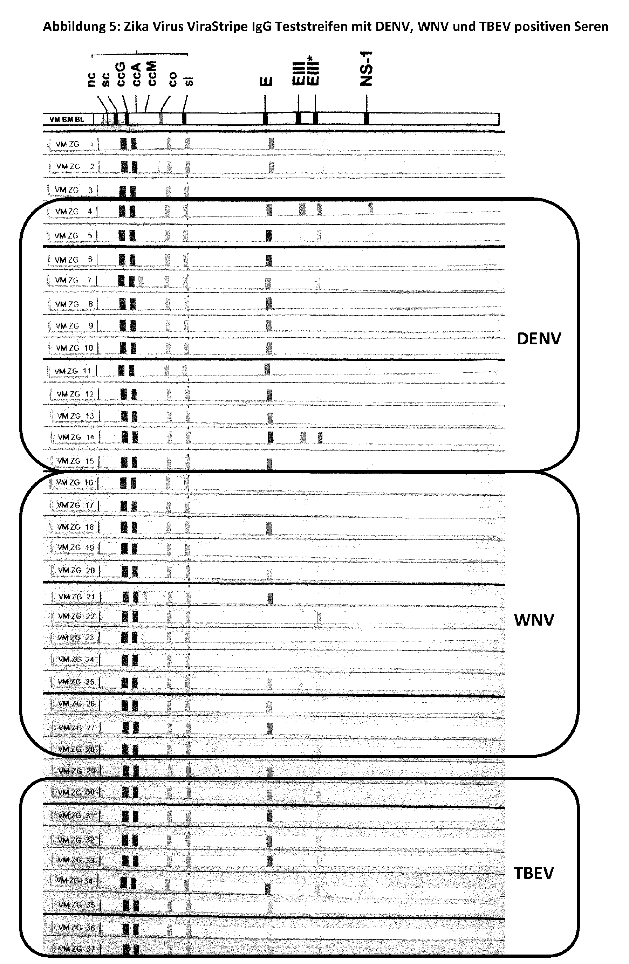

Insbesondere das E-Antigen zeigt eine hohe Kreuzreaktivität mit DENV und TBEV positiven Seren (siehe Abbildung 5). Durch Kombination mit dem NS-1 Antigen und mit geeigneten Interpretationskriterien bleibt die Testspezifität jedoch erhalten.

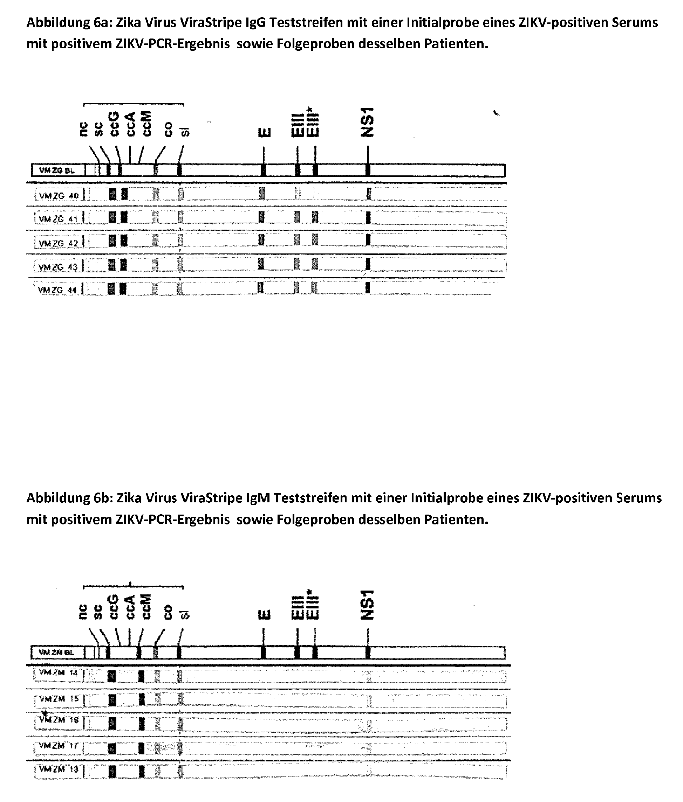

Die Analyse von Seren von einem Patienten mit klinisch und diagnostisch gesicherter ZIKV Infektion zeigt, dass IgM Antikörper gegen NS1 gebildet werden können und im Verlauf der Infektion bezüglich der Reaktivität ansteigen und anschließend wieder absinken (siehe Abbildung 6). Überraschenderweise ergibt sich aus der Analyse der IgG Antikörper, dass zunächst vornehmlich IgG Antikörper gegen die Proteine E und NS1 nachgewiesen werden können und später im Verlaufe der Infektion bzw. Erkrankung erst gegen die Fragmente des Envelope (E) - Proteins.

### Referenzen/ Literaturangaben

Calvet G et al. Lancet Infect Dis. 2016 Feb 17. S1473-3099(16)00095-5.
Charrel RN et al. 2016 Feb 10. Bulletin of the World Health Organization, Article ID: BLT.16.171207
Cauchemez S et al. Lancet. 2016 Mar 15. S0140-6736(16)00651-6.
Dick et al. Trans R Soc Trop Med Hyg. 1952 Sep;46(5):509-20.
Enfissi A et al., Lancet. 2016 Jan 16;387(10015):227-8.
Faye O et al. 2014. PLoS Negl Trop Dis. 2014 Jan 9;8(1):e2636.
Foy BD et al. Emerg Infect Dis. 2011 May;17(5):880-2.
Gyurech D et al., 2016., Swiss Med Wkly. 2016 Feb 9;146:w14296.
Haddow AD et al. PLoS Negl Trop Dis. 2012;6(2):e1477.
Li L et al., Science. 2008 Mar 28;319(5871):1830-4.
MansuyJM et al. Lancet Infect Dis. 2016 Apr;16(4):405.
McCarthy M. BMJ. 2016 Feb 4;352.
Mlakar J et al. N Engl J Med. 2016 Mar 10;374(10):951-8.
Musso D et al. Emerg Infect Dis. 2015 Feb;21(2):359-61.
Musso D. Emerg Infect Dis. 2015 Oct;21(10):1887.
Sirohi D et al. Science. 2016 Mar 31.10.1126/scienceaaf5316.

## Patentansprüche

1. Träger zum Nachweis von Zika Virus,
wobei der Träger mindestens zwei Antigene umfasst,
wobei die Antigene ausgewählt sind aus
a) einem Antigen umfassend SEQ ID NR 1,
b) einem Antigen umfassend SEQ ID NR 2,
c) einem Antigen umfassend SEQ ID NR 3 und
d) einem Antigen umfassend SEQ ID NR 4,
wobei jedes dieser mindestens zwei Antigene als Variante vorliegen kann.

2. Träger nach Anspruch 1, der mindestens drei Antigene wie in Anspruch 1 a bis d definiert umfasst, wobei jedes dieser mindestens drei Antigene als Variante vorliegen kann.

3. Träger nach Anspruch 1 oder 2, der mindestens vier Antigene wie in Anspruch 1 a bis d definiert umfasst, wobei jedes dieser mindestens vier Antigene als Variante vorliegen kann.

4. Träger nach Anspruch 1, umfassend zwei Antigene umfassend SEQ ID NR 1 bzw. SEQ ID NR 4, wobei jedes dieser mindestens zwei Antigene als Variante vorliegen kann.

5. Träger nach Anspruch 1, umfassend zwei Antigene umfassend SEQ ID NR 2 bzw. SEQ ID NR 3, wobei jedes dieser mindestens zwei Antigene als Variante vorliegen kann.

6. Träger nach Anspruch 2, umfassend drei Antigene umfassend SEQ ID NR 1, SEQ ID NR 2 bzw. SEQ ID NR 4, wobei jedes dieser mindestens drei Antigene als Variante vorliegen kann.

7. Träger nach Anspruch 2, umfassend drei Antigene umfassend SEQ ID NR 1, SEQ ID NR 3 bzw. SEQ ID NR 4, wobei jedes dieser mindestens drei Antigene als Variante vorliegen kann.

8. Träger nach Anspruch 2, umfassend drei Antigene umfassend SEQ ID NR 2, SEQ ID NR 3 bzw. SEQ ID NR 4, wobei jedes dieser mindestens drei Antigene als Variante vorliegen kann.

9. Träger nach einem der Ansprüche 1 bis 8 , der mindestens ein weiteres aus dem Zika Virus abgeleitetes Antigen umfasst.

10. Träger nach einem der Ansprüche 1 bis 8, der mindestens ein weiteres aus einem vom Zika Virus verschiedenen Pathogen abgeleitetes Antigen umfasst.

11. Träger nach Anspruch 10, wobei das mindestens eine vom Zika Virus verschiedene Pathogen ausgewählt ist aus einer Gruppe, die DENV, WNV, YFV, TBEC, SLV, JEV, CHIKV, EBV umfasst.

12. Verfahren zum Nachweis einer Zika Virus Infektion umfassend
- das Bereitstellen eines Trägers gemäß einem der Ansprüche 1 bis 11;
- Inkubieren einer biologischen Probe, die möglicherweise Anti-ZIKV-Antikörper enthält, mit dem Träger unter Bedingungen, die eine Ausbildung mindestens eines Antigen-Antikörper-Komplexes erlauben;
- Bestimmen der Anwesenheit von Anti-ZIKV-Antikörpern in der Probe durch Nachweis mindestens eines Antigen-Antikörper-Komplexes.

13. Verwendung von mindestens zwei Antigenen oder Varianten dieser Antigene gemäß Anspruch 1 zum Nachweis von anti-Zika Virus Antikörpern.

14. Verwendung nach Anspruch 13, wobei der Nachweis eine Abgrenzung gegen von Zika Virus verschiedene Pathogene ermöglicht.

15. Verwendung nach Anspruch 14, wobei die vom Zika Virus verschiedenen Pathogene ausgewählt sind aus DENV, WNV, YFV, TBEC, SLV, JEV, CHIKV, EBV.

16. Verwendung nach Anspruch 13, 14 oder 15, umfassend die Bestimmung des Infektionsstatus.

17. Verwendung nach einem der Ansprüche 13 bis 16, umfassend die Bestimmung der Immunglobulinklasse der nachgewiesenen Antikörper.

18. Testkit umfassend Antigene oder Varianten dieser Antigene wie in einem der Ansprüche 1 bis 11 definiert.

19. Testkit nach Anspruch 18 umfassend eine Anweisung zur Auswertung der Testergebnisse, welche mit dem Testkit gewonnen werden.
